# EUROPÄISCHE PATENTSCHRIFT

(11) **EP 3 310 248 B1**
(45) Veröffentlichungstag und Bekanntmachung des Hinweises auf die Patenterteilung: **18.09.2019**
(21) Anmeldenummer: 16719836.5
(22) Anmeldetag: 26.04.2016
(51) Int. Cl.: A61B 5/00, A61B 5/11, A61B 5/18

(54) **VERFAHREN UND VORRICHTUNG ZUM UNTERSCHEIDEN VON BLINZELEREIGNISSEN UND INSTRUMENTENBLICKEN UNTER VERWENDUNG EINER AUGENÖFFNUNGSWEITE**
METHOD AND DEVICE FOR DISTINGUISHING BLINKING EVENTS AND INSTRUMENT GLANCES BY USING AN EYE OPENING WIDTH
PROCÉDÉ ET DISPOSITIF POUR DISTINGUER DES ÉVÉNEMENTS DE CLIGNEMENT ET DES COUPS D'OEIL SUR DES INSTRUMENTS PAR L'UTILISATION D'UNE LARGEUR D'OUVERTURE DE L'OEIL

(30) Priorität: 22.06.2015 DE 102015211444
(43) Veröffentlichungstag der Anmeldung: 25.04.2018
(73) Patentinhaber: Robert Bosch GmbH, 70442 Stuttgart (DE)
(72) Erfinder: WULF, Felix, 71636 Ludwigsburg (DE); VANDOMMELE, Tjark, 70499 Stuttgart (DE)
(86) Internationale Anmeldenummer: PCT/EP2016/059233
(87) Internationale Veröffentlichungsnummer: WO 2016/206831

(56) Entgegenhaltungen:
- EP-A1- 2 351 524
- JP-A- 2002 029 279
- JP-A- 2003 000 571
- JP-A- 2008 197 821

## Beschreibung

### Stand der Technik

Die Erfindung geht aus von einer Vorrichtung oder einem Verfahren nach Gattung der unabhängigen Ansprüche. Gegenstand der vorliegenden Erfindung ist auch ein Computerprogramm.

Schläfrigkeit und Sekundenschlaf am Steuer führen häufig zu gefährlichen Situationen oder Unfällen.

Die Schläfrigkeit eines Fahrers eines Fahrzeugs kann indirekt aus seinem Fahrverhalten geschätzt werden.

In der Literatur werden Zusammenhänge zwischen den Eigenschaften von Blinzelereignissen und der Schläfrigkeit diskutiert.

Die WO 2014031042 A1 beschreibt ein Fitten eines Augenöffnungssignals mit vordefinierten modellierten Signalen, um Blinzelereignisse zu erkennen und daraus Rückschlüsse auf die Aufmerksamkeit des Fahrers zu ziehen.

Die Druckschrift JP 2003 000571 A offenbart eine Vorrichtung und ein Verfahren zur Schätzung des Müdigkeitsgrades.

Die Druckschrift JP 2002 029279 A offenbart eine Vorrichtung zur Bestimmung einer Größe der Wachheitsabnahme.

Die Druckschrift JP 2008197821 A offenbart eine Vorrichtung zur Schläfrigkeitswarnung und ein Warnverfahren für eine solche Vorrichtung.

Die Druckschrift EP 2 351 524 A1 offenbart eine Vorrichtung zur Bestimmung einer Schläfrigkeit und ein entsprechendes Programm.

### Offenbarung der Erfindung

Vor diesem Hintergrund werden mit dem hier vorgestellten Ansatz ein Verfahren zum Unterscheiden von Blinzelereignissen und Instrumentenblicken unter Verwendung einer Augenöffnungsweite, weiterhin eine Vorrichtung, die dieses Verfahren verwendet, sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Um eine Aussage über eine Müdigkeit beziehungsweise Schläfrigkeit einer Person unter Verwendung von Blinzelereignissen zu erhalten, ist es erforderlich, nur tatsächliche Blinzelereignisse der Person auszuwerten. Willentlich von der Person ausgeführte Augenbewegungen, die zu reflexartigen Lidbewegungen führen, wie das Senken der Blickrichtung beispielsweise auf Instrumente einer Instrumententafel, weisen jedoch ähnliche Anfangscharakteristiken der Lidbewegung auf, wie Blinzelereignisse.

Daher wird bei dem hier vorgestellten Ansatz eine Unterscheidung zwischen einem Blinzelereignis und einem gesenkten Blick basierend auf einer Dauer des Ereignisses durchgeführt, da ein Blinzelereignis nach kurzer Zeit beendet sein sollte.

Es wird ein Verfahren zum Unterscheiden von Blinzelereignissen und Instrumentenblicken unter Verwendung einer Augenöffnungsweite vorgestellt, wobei die Augenöffnungsweite einen aktuell erfassten Abstand zwischen den Augenlidern eines Auges repräsentiert, wobei das Verfahren die folgenden Schritte aufweist:
Ermitteln eines Blinzelereignisses unter Verwendung zumindest eines Blinzelgrenzwerts, wobei das Blinzelereignis ermittelt wird, wenn ein, einen Wert der Augenöffnungsweite abbildender Öffnungsweitenwert kleiner ist, als der Blinzelgrenzwert; und
Bestimmen eines Instrumentenblicks unter Verwendung einer Maximalblinzeldauer, wobei der Instrumentenblick bestimmt wird, wenn das erkannte Blinzelereignis als länger als die Maximalblinzeldauer dauernd erkannt wird, insbesondere wobei die Schritte des Verfahrens erneut ausgeführt werden, wenn nachfolgend auf das Bestimmen des Instrumentenblicks der Öffnungsweitenwert größer als ein Offengrenzwert ist.

Unter einem Blinzelereignis kann ein Blinzeln, ausgehend von einem geöffneten Auge, über ein weitgehendes Schließen des Auges bis zu einem erneuten Öffnen des Auges, verstanden werden. Ein Instrumentenblick kann ein, auf eine Geschwindigkeitsanzeige und/oder eine andere Anzeige eines Fahrzeugs, gesenkter Blick verstanden werden. Insbesondere kann der Instrumentenblick als Blick auf ein frei programmierbares Kombiinstrument verstanden werden. Eine Augenöffnungsweite kann auch als Augenschlussweite verstanden werden. Ein gesenkter Blick, der kürzer als die Maximalblinzeldauer ist, wird als Blinzelereignis bewertet.

Im Schritt des Ermittelns kann ein Mittelblinzelereignis ermittelt werden, wenn der Öffnungsweitenwert kleiner ist, als ein Mittelblinzelgrenzwert. Weiterhin kann ein Tiefblinzelereignis ermittelt werden, wenn der Öffnungsweitenwert kleiner ist, als ein Tiefblinzelgrenzwert. Ebenso kann ein Tiefblinzelereignis ermittelt werden, wenn ein auf das Ermitteln des Mittelblinzelereignisses nachfolgender Öffnungsweitenwert kleiner als der Tiefblinzelgrenzwert ist. Als ein Mittelblinzelereignis wird vorliegend ein Blinzelereignis mit einer mittleren Augenöffnungsweite verstanden, wogegen ein Tiefblinzelereignis als ein Blinzelereignis mit einer niedrigen Augenöffnungsweite, d. h. mit einer Augenöffnungsweite, die kleiner als die mittlere Augenöffnungsweite ist, beziehungsweise mit geschlossenem Auge verstanden wird. Durch eine Unterscheidung zwischen Mittelblinzelereignissen und Tiefblinzelereignissen kann eine Analyse der aktuellen Schläfrigkeit einer Person verbessert werden.

Der Instrumentenblick kann bestimmt werden, wenn das Mittelblinzelereignis länger als die Maximalblinzeldauer ermittelt wird. Alternativ oder ergänzend kann kein Instrumentenblick bestimmt werden, wenn das Tiefblinzelereignis länger als die Maximalblinzeldauer ermittelt wird. Durch die Trennung zwischen Mittelblinzelereignissen und Tiefblinzelereignissen wird eine Unterscheidung der Instrumentenblicke vereinfacht.

Das Verfahren kann einen Schritt des Speicherns aufweisen, in dem ein zeitlicher Verlauf des Öffnungsweitenwerts als ein Blinzelereignisverlauf gespeichert wird, wenn nachfolgend auf das Ermitteln des Blinzelereignisses der Öffnungsweitenwert größer als der Blinzelgrenzwert ist. Nachfolgend auf den Schritt des Speicherns können die Schritte des Verfahrens erneut ausgeführt werden. Im Schritt des Speicherns können Öffnungsweitenwerte innerhalb eines bestimmten Zeitraums gespeichert werden. Das Speichern kann rückwirkend stattfinden. Der Startzeitpunkt des zu speichernden Zeitraums kann vor einem Beginn de Blinzelereignisses liegen. Durch das Speichern des Blinzelereignisverlaufs kann das Blinzelereignis weiter ausgewertet werden.

Das Verfahren kann einen Schritt des Interpolierens aufweisen, in den die Öffnungsweitenwerte des Blinzelereignisverlaufs zu einer Kurve verbunden werden. Durch eine Kurve kann das Blinzelereignis unterbrechungsfrei ausgewertet werden.

Die Kurve kann unter Verwendung einer vorbestimmten Kurvenform verbessert oder approximiert werden. Eine Berechnung der Kurve kann durch eine erwartete Kurvenform vereinfacht werden. Die Kurvenform kann beispielsweise ein standardisiertes Blinzelereignis abbilden, das unter Verwendung von einem oder mehreren Blinzelparametern an das tatsächliche Blinzelereignis angepasst werden kann.

Im Schritt des Interpolierens kann zumindest ein Abweichungswert mindestens eines der Öffnungsweitenwerte von der Kurve bestimmt werden. Ein Abweichungswert kann eine Differenz zwischen einem Wert eines zu einem Öffnungsweitenwert korrespondierenden Kurvenpunkts und dem Öffnungsweitenwert repräsentieren. Die Kurve kann schrittweise optimiert werden. Dabei kann der Fehler über zumindest eine Mehrheit der gespeicherten Öffnungsweitenwerte gemittelt minimiert werden.

Das Verfahren kann einen Schritt des Plausibilisierens aufweisen, in dem der Blinzelereignisverlauf verworfen wird, wenn der zumindest eine Abweichungswert oder ein davon abgeleiteter Wert wie beispielsweise ein Mittelwert größer als ein Abweichungsgrenzwert ist. Durch ein Aussondern von ungewöhnlichen Blinzelereignissen kann die Schläfrigkeit der Person mit guter Wahrscheinlichkeit bestimmt werden.

Das Verfahren kann einen Schritt des Veränderns aufweisen, in dem der Blinzelgrenzwert und alternativ oder ergänzend der Offengrenzwert unter Verwendung eines Bezugsniveaus für die Augenöffnungsweite angepasst wird. Dabei repräsentiert das Bezugsniveau die Augenöffnungsweite, wenn kein Blinzelereignis und alternativ oder ergänzend Instrumentenblick vorliegt. Ebenso kann der Mittelblinzelgrenzwert und/oder Tiefblinzelgrenzwert unter Verwendung des Bezugsniveaus angepasst werden. Damit funktioniert das hier vorgestellte Verfahren unabhängig von Faktoren, die die Augenöffnungsweite außerhalb von Blinzelereignissen und/oder Instrumentenblicken beeinflussen.

Dieses Verfahren kann beispielsweise in Software oder Hardware oder in einer Mischform aus Software und Hardware beispielsweise in einem Steuergerät implementiert sein.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung kann die der Erfindung zugrunde liegende Aufgabe schnell und effizient gelöst werden.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele der Erfindung sind in den Zeichnungen dargestellt und in der nachfolgenden Beschreibung näher erläutert. Es zeigt:
Fig. 1 ein Blockschaltbild einer Vorrichtung zum Unterscheiden von Blinzelereignissen und Instrumentenblicken gemäß einem Ausführungsbeispiel;
Fig. 2 eine Darstellung einer Architektur eines Gesamtsystems zum Überwachen der Schläfrigkeit eines Fahrers eines Fahrzeugs gemäß einem Ausführungsbeispiel;
Fig. 3 ein Zustandsdiagramm für ein Erkennen von Blinzelereignissen gemäß einem Ausführungsbeispiel;
Fig. 4 eine Darstellung einer Augenöffnungskurve eines Blinzelereignisses gemäß einem Ausführungsbeispiel; und
Fig. 5 ein Ablaufdiagramm eines Verfahrens zum Unterscheiden von Blinzelereignissen und Instrumentenblicken gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

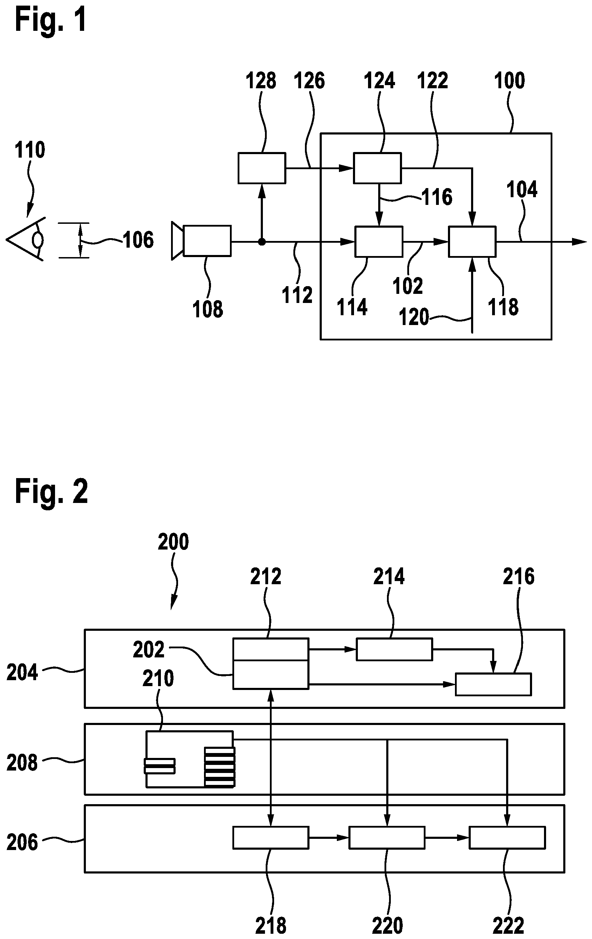

Fig. 1 zeigt ein Blockschaltbild einer Vorrichtung 100 zum Unterscheiden von Blinzelereignissen 102 und Instrumentenblicken 104 gemäß einem Ausführungsbeispiel. Dabei werden die Blinzelereignisse 102 und Instrumentenblicke 104 unter Verwendung einer Augenöffnungsweite 106 unterschieden. Die Augenöffnungsweite 106 wird hier durch ein Erfassungssystem 108 an einem oder beiden Augen 110 eines Fahrers eines Fahrzeugs erfasst und in einem Öffnungsweitenwert 112 abgebildet. Die Augenöffnungsweite 106 repräsentiert einen aktuellen Abstand zwischen den Augenlidern des Auges 110. Der Öffnungsweitenwert 112 wird von der Vorrichtung 100 an einem Eingang der Vorrichtung 100 eingelesen. In der Vorrichtung 100 wird der Öffnungsweitenwert 112 in einer Ermittlungseinrichtung 114 verwendet, um unter Verwendung zumindest eines Blinzelgrenzwerts 116 ein Blinzelereignis 102 zu ermitteln. Dabei wird das Blinzelereignis ermittelt, wenn der Öffnungsweitenwert 112 kleiner als der Blinzelgrenzwert 116 ist.

In einer Bestimmungseinrichtung 118 wird ein Instrumentenblick 104 unter Verwendung einer Maximalblinzeldauer 120 bestimmt. Dabei wird der Instrumentenblick 104 bestimmt, wenn das erkannte Blinzelereignis 102 länger als die Maximalblinzeldauer 120 erkannt wird. Wenn nachfolgend auf das Bestimmen des Instrumentenblicks 104 der Öffnungsweitenwert 112 größer als ein Offengrenzwert 122 ist, wird in der Ermittlungseinrichtung 114 das nächste Blinzelereignis bestimmt.

In einem Ausführungsbeispiel weist die Vorrichtung 100 eine Veränderungseinrichtung 124 auf. In der Veränderungseinrichtung 124 wird der Blinzelgrenzwert 116 und alternativ oder ergänzend der Offengrenzwert 122 unter Verwendung eines Bezugsniveaus 126 für die Augenöffnungsweite 106 angepasst. Das Bezugsniveau 126 repräsentiert die Augenöffnungsweite 104, wenn kein Blinzelereignis 102 und alternativ oder ergänzend kein Instrumentenblick 104 vorliegt. Das Bezugsniveau 126 wird von einer Vorrichtung 128 zum Bereitstellen des Bezugsniveaus 126 unter Verwendung des Öffnungsweitenwerts 112 bereitgestellt.

Basierend auf Daten einer Videokamera 108 kann ein momentaner Öffnungsgrad 106 der Augen 110 erkannt werden. Dazu werden entsprechende Bildverarbeitungsalgorithmen verwendet. Dabei kann jeweils für beide Augen 110 ein Augenöffnungsniveau 126 detektiert werden.

Aus den Augenöffnungsgraden 106 zweier Augen 110 kann ein gemeinsames Augenöffnungsniveau 126 berechnet werden. Die Berechnung des momentanen Augenöffnungsniveaus 126 kann unter Verwendung von Savitzky-Golay Filtern erfolgen.

Durch den hier vorgestellten Ansatz kann die Schläfrigkeit beziehungsweise ein Schläfrigkeitszustand eines Fahrers mit Hilfe von Lidschlussdaten 112, beispielsweise von einer Kamera 108, klassifiziert beziehungsweise präzise eingeschätzt werden. Es wird somit beispielhaft vorgeschlagen, "gefittete" Kurven zur Erhöhung der Erkennungsgüte und Unterscheidung von zuvor detektierten Blinzlern zu verwenden.

Fig. 2 zeigt eine Darstellung einer Architektur eines Gesamtsystems 200 zum Überwachen der Schläfrigkeit eines Fahrers eines Fahrzeugs gemäß einem Ausführungsbeispiel. Das hier vorgestellte Unterscheiden 202 von Blinzelereignissen und Instrumentenblicken ist ein Bestandteil des Gesamtsystems 200.

Das Gesamtsystem 200 weist drei Hauptbestandteile 204, 206, 208 auf. Der erste Hauptbestandteil 204 wird als Schläfrigkeitsklassifizierung bezeichnet. Der zweite Hauptbestandteil 206 wird als Sekundenschlaferkennung bezeichnet. Der dritte Hauptbestandteil 208 umfasst von der Schläfrigkeitsklassifizierung 204 und der Sekundenschlaferkennung 206 gemeinsam genutzte Module 210.

Die hier vorgestellte Unterscheidung 202 ist Bestandteil der Schläfrigkeitsklassifizierung 204 und kann als Blinzelereignisdetektion 202 bezeichnet werden.

Die Module 210 können als Augenschlussvorverarbeitung 210 bezeichnet werden. Die Augenschlussvorverarbeitung 210 umfasst eine Erfassung des Augenschlusses rechts und links, eine Filterung des Augenschlusses, eine Geschwindigkeitserfassung des Augenschlusses, eine acc des Augenschlusses, eine Bereitstellung eines Bezugsniveaus und eine Validierung.

Die Augenschlussvorverarbeitung 210 gibt einen momentanen Augenschluss, eine Augenschlussgeschwindigkeit und das Bezugsniveau aus.

In der Schläfrigkeitsklassifizierung 204 werden diese Werte in der Blinzelereignisdetektion 202 verwendet und Blinzelereignisse an eine Blinzelmerkmalsberechnung 212 weitergeleitet.

Die Blinzelmerkmalsberechnung 212 gibt Blinzelmerkmale an eine persönliche Blinzelmerkmalserkennung 214 und ein Modul 216 zur Schläfrigkeitsklassifizierung aus. Das Modul liest von der Blinzelmerkmalserkennung 214 ein persönliches Blinzelverhalten ein und gibt ein Schläfrigkeitsniveau aus.

In der Sekundenschlaferkennung 206 werden die Werte in einer persönlichen Augenschlusserkennung 218, einer Augenschlusserkennung 220 und einem Modul 222 zur Sekundenschlaferkennung verwendet.

Die persönliche Augenschlusserkennung 218 gibt ein persönliches Offenaugenniveau und ein persönliches Geschlossenaugenniveau aus. Beide werden von der Augenschlusserkennung 220 verwendet, um einen binären Augenoffenwert für das Modul 222 bereitzustellen. Das Modul 222 gibt Sekundenschlafereignisse aus.

Der hier vorgestellte Ansatz stellt eine Verbesserung der Erkennungsgüte von Blinzelfeatures auf Basis von zuvor vorgefilterten Augenöffnungsdaten dar. Die hier vorgestellte, sogenannte Blinzelereignisdetektion (Blink Event Detection, BED) 202 gliedert sich in ein Gesamtsystem 200 zur Erkennung von Schläfrigkeit und/oder Sekundenschlaf ein. Das Gesamtsystem 200 umfasst ein Eye Closure Preprocessing 204. Ein momentanes Augenöffnungsniveau (EON) kann unter Verwendung eines Algorithmus berechnet werden. Das Gesamtsystem 200 kann eine Sekundenschlafdetektion 206 umfassen.

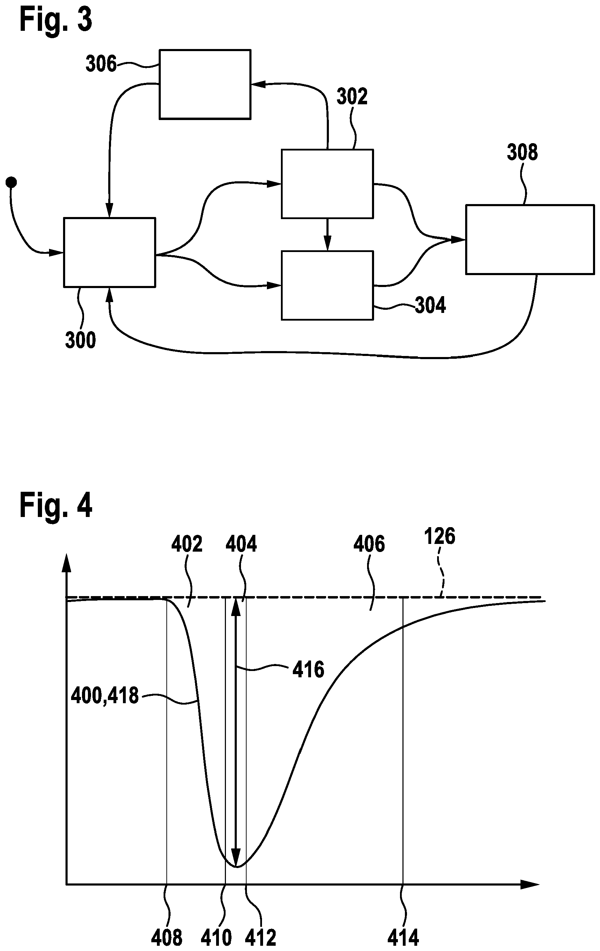

Fig. 3 zeigt ein Zustandsdiagramm für ein Erkennen von Blinzelereignissen gemäß einem Ausführungsbeispiel. In dem Zustandsdiagramm sind verschiedene Erkennungszustände in Abhängigkeit von einer Augenöffnungsweite dargestellt. Ausgehend von einem Offenzustand 300 wird die momentane Augenöffnungsweite mit einem Mittelblinzelgrenzwert und einem Tiefblinzelgrenzwert verglichen. Wenn die Augenöffnungsweite kleiner als der Mittelblinzelgrenzwert ist, wird ein Mittelblinzelzustand 302 erkannt. Wenn die Augenöffnungsweite kleiner als der Tiefblinzelgrenzwert ist, wird ein Tiefblinzelzustand 304 erkannt.

Wenn der Mittelblinzelzustand 302 erkannt ist und die Augenöffnungsweite wird kleiner als der Tiefblinzelgrenzwert, so wird der Tiefblinzelzustand 304 erkannt.

Wenn der Mittelblinzelzustand 302 länger als eine Maximalblinzeldauer erkannt wird, wird ein Instrumentenblickzustand 306 erkannt.

Wenn nach dem Instrumentenblickzustand 306 die Augenöffnungsweite größer als ein Offengrenzwert wird, wird wieder der Offenzustand 300 erkannt.

Wenn der Mittelblinzelzustand 302 erkannt ist, und die Augenöffnungsweite wird größer als der Offengrenzwert, wird ein Blinzelende 308 erkannt und ein Verlauf der Augenöffnungsweite während des Blinzelereignisses wird gespeichert.

Ebenso wird das Blinzelende 308 erkannt, wenn der Tiefblinzelzustand 304 erkannt ist und die Augenöffnungsweite wird größer als der Mittelblinzelgrenzwert. Dann wird ebenfalls der Verlauf der Augenöffnungsweite während des Blinzelereignisses gespeichert.

Nach dem Blinzelende 308 weist das Auge wieder den Offenzustand 300 auf.

Es wird ein System zur robusten Detektion von Blinzelereignissen und zur Erhöhung der Qualität des Sensorsignals vorgestellt.

Ein Ausführungsbeispiel des hier vorgestellten Algorithmus zur Erkennung von Blinzelereignissen und zur Verbesserung der Qualität der Eingangsgrößen weist einen Schritt des Detektierens, einen Schritt des Fittens und einen Schritt des Berechnens auf. Dieses Ausführungsbeispiel ist anhand der Figuren 3 und 4 beschrieben.

Im Schritt des Detektierens werden potenzielle Blinzelereignisse mit Hilfe von vorbestimmten Grenzwerten der Augenöffnung detektiert.

Dabei werden Blinzelereignisse von Instrumentenblicken unterschieden. Instrumentenblicke haben keinen Bezug zur Schläfrigkeit des Fahrers. Sie können daher im weiteren Verlauf verworfen werden. Potenzielle Blinzelereignisse werden mithilfe einer State-Machine und drei verschiedenen Grenzwerten identifiziert. Dabei werden ein oberer Grenzwert, ein mittlerer Grenzwert und ein unterer Grenzwert verwendet. Alle drei verwendeten Grenzwerte (High, Mid und Low) können als prozentualer Betrag des Augenöffnungsniveaus (EON) definiert werden. Beispielsweise kann der obere Grenzwert (High) stets 100% des Augenöffnungsniveaus betragen. Der mittlere Grenzwert (Mid) kann stets 70% des Augenöffnungsniveaus betragen. Der untere Grenzwert (Low) kann stets 20 % des Augenöffnungsniveaus betragen. Die absoluten Werte sind daher abhängig von der momentanen Augenöffnung des Fahrers werden dadurch adaptiv an die Gegebenheiten angepasst.

In Fig. 3 ist das vorgeschlagene Zustandsdiagramm zur Erkennung von Blinzelereignissen mit großer und kleiner Amplitude sowie von Instrumentenblicken dargestellt, wobei hier die Anzeigeinstrumente, wie beispielsweise ein frei programmierbares Kombiinstrument (FPK) als Tacho bezeichnet werden. Je nachdem, welchen aktuellen Wert ein Augenöffnungssignal (EC) aufweist, werden die verschiedenen Zustände 300, 302, 304, 306, 308 unterschieden.

Zur Unterscheidung von Blinzelereignissen mit kleiner Amplitude und Instrumentenblicken wird eine Maximaldauer (Max_Dur) für den Zustand "Midblink" beziehungsweise ein mittleres Blinzelereignis eingeführt. Diese kann z. B. auf 0,5 s eingestellt werden.

Sobald der Zustand 308 "Save" erreicht wird, werden die Rohdaten des Blinzelereignisses zur weiteren Verarbeitung gespeichert. Dazu werden die Augenöffnungsdaten in einem ausreichend großen Fenster (z. B. +- 2 s um den Punkt mit dem niedrigsten Augenöffnungsgrad) ausgeschnitten und gespeichert. Anschließend geht die State-Machine sofort zurück in den Zustand 300 "Open".

Fig. 4 zeigt eine Darstellung einer Augenöffnungskurve 400 eines Blinzelereignisses gemäß einem Ausführungsbeispiel. Die Augenöffnungskurve 400 ist in einem Diagramm dargestellt, das auf seiner Abszisse eine Zeit und auf seiner Ordinate eine Lidöffnung angetragen hat. Die Augenöffnungskurve 400 repräsentiert eine Interpolation eines Verlaufs von Einzelwerten einer Augenöffnungsweite beziehungsweise Lidöffnung. Der Verlauf ist, wie in Fig. 3 dargestellt, ansprechend auf ein Erkennen eines Endes des Blinzelereignisses rückwirkend abgespeichert worden.

Das Blinzelereignis weist eine Schließphase 402, eine Schlussphase 404 und eine Öffnungsphase 406 auf. Die Schlussphase 404 kann auch als Plateauphase 404 bezeichnet werden. Die Schließphase 402 erstreckt sich von einem Blinzelstart 408 bis zu einem Plateaustart 410. Die Schlussphase 404 erstreckt sich von dem Plateaustart 410 bis zu einem Plateauende 412. Die Öffnungsphase 406 erstreckt sich von dem Plateauende 412 bis zu einem Blinzelende 414. Innerhalb der Schlussphase 404 weist die Augenöffnungskurve 400 ihr lokales Minimum beziehungsweise ihre maximale negative Amplitude 416 während des Blinzelereignisses auf.

Vor dem Blinzelstart 408 und nach dem Blinzelende 414 erreicht die Augenöffnungskurve 400 als maximalen Wert ein momentanes Bezugsniveau 126 der Augenöffnungsweite, das beispielsweise von einer momentanen Lichtsituation abhängt.

Falls der zum Einsatz kommende Sensor noch eine unzureichende Genauigkeit oder Framerate aufweisen sollte, können die, wie in Fig. 3 beschrieben, erkannten potenziellen Blinzelereignisse in einem weiteren Schritt verfeinert werden. Dazu wird für alle im ersten Schritt detektierten Fenster ein Fitting mit einer vordefinierten Kurve 418 durchgeführt. Diese Kurve 418 bildet den idealen Verlauf eines Blinzelereignisses nach.

Im Schritt des Fittens werden diese Blinzelereignisse mit vordefinierten Kurven 418 gefittet, um eine niedrige Bildwiederholfrequenz des verwendeten Bildsensors auszugleichen, was etwa so wirkt, wie wenn die Framerate des Sensors erhöht wäre.

In Fig. 4 ist ein idealisierter Verlauf 400 eines Blinzelereignisses dargestellt. Die Kurve 418 weist hier beispielsweise vor dem Blinzelstart 408 die Form einer horizontalen Gerade auf. In der Schließphase 402 weist die Kurve 418 die Form einer abfallenden Kurve, beispielsweise fünfter Ordnung auf. In der Plateauphase 404 weist die Kurve 418 die Form einer horizontalen Gerade auf. In der Öffnungsphase 406 weist die Kurve 418 die Form einer steigenden Kurve, beispielsweise fünfter Ordnung auf. Nach dem Blinzelende 414 weist die Kurve 418 beispielsweise die Form einer horizontalen Gerade auf.

Dabei wird die Kurve 418 unter der Randbedingung an Verlauf von Einzelwerten angepasst, dass die sich ergebende Gesamtkurve 400 sowie ihre erste Ableitung im gesamten betrachteten Fenster einen stetigen Verlauf aufweist.

Der Anfangspunkt und der Endpunkt der Kurve 400 ergeben sich hier aus den Grenzen des im vorherigen Schritt detektierten Fensters. Die anderen Zeitpunkte 408, 410, 412, 414 ergeben sich in einer Optimierungsphase des Fittings. Für die einzelnen zeitlichen Bereiche ergeben sich also unterschiedliche Koeffizienten der Kurventeile 402, 404, 406. Diese können zum einen analytisch weiterverarbeitet werden, es kann aber auch eine beliebig feine Interpolation der Kurve 418 berechnet werden. Die hier dargestellte Methode ist also durch die Interpolation in der Lage, die Sample-Rate des Sensorsignals zu erhöhen.

Das Fitting wird in einem Ausführungsbeispiel so optimiert, dass eine Fehlerfunktion minimal wird. Hier eignet sich beispielsweise die Summe der quadratischen Abweichung der Messwerte mit der gefitteten Kurve 418, die minimal sein sollte.

Im Schritt des Berechnens wird ein Qualitätsmaß für die Güte des Fittings berechnet.

Die im vorherigen Schritt berechnete Fehlerfunktion kann in einem weiteren optionalen Schritt dazu verwendet werden, Blinzelereignisse mit einem unplausiblen Verlauf 400, also die nicht dem idealen Verlauf entsprechen, aus der weiteren Bearbeitung auszuschließen. Hierzu kann das Resultat der Fehlerfunktion mit einem zuvor definierten Grenzwert verglichen werden. Wird dieser für ein spezifisches Blinzelereignis überschritten, wird es in den weiteren Berechnungen zur Klassifikation der Schläfrigkeit nicht weiter herangezogen.

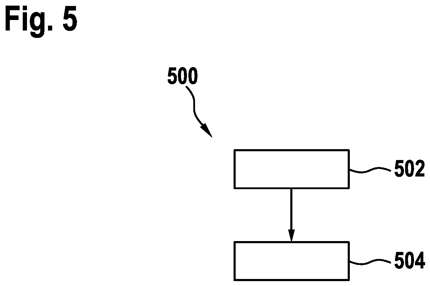

Fig. 5 zeigt ein Ablaufdiagramm eines Verfahrens 500 zum Unterscheiden von Blinzelereignissen und Instrumentenblicken gemäß einem Ausführungsbeispiel. Das Verfahren kann beispielsweise auf einer Vorrichtung, wie sie in Fig. 1 dargestellt ist, ausgeführt werden. Das Verfahren 500 weist einen Schritt 502 des Ermittelns und einen Schritt 504 des Bestimmens auf.

Im Schritt 502 des Ermittelns wird ein Blinzelereignis unter Verwendung zumindest eines Blinzelgrenzwerts ermittelt. Dabei wird das Blinzelereignis ermittelt, wenn ein, einen Wert einer Augenöffnungsweite abbildender Öffnungsweitenwert kleiner ist, als der Blinzelgrenzwert. Die Augenöffnungsweite repräsentiert einen aktuell erfassten Abstand zwischen den Augenlidern eines Auges.

Im Schritt 504 des Bestimmens wird ein Instrumentenblick unter Verwendung einer Maximalblinzeldauer bestimmt. Dabei wird der Instrumentenblick bestimmt, wenn das erkannte Blinzelereignis als länger als die Maximalblinzeldauer dauernd erkannt wird.

In einem Ausführungsbeispiel werden die Schritte 502, 504 des Verfahrens erneut ausgeführt, wenn nachfolgend auf das Bestimmen 504 des Instrumentenblicks der Öffnungsweitenwert größer als ein Offengrenzwert ist.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (500) zum Unterscheiden von Blinzelereignissen (102) und Instrumentenblicken (104) mit Hilfe einer Vorrichtung unter Verwendung einer Augenöffnungsweite (106), wobei ein Instrumentenblick (104) einem gesenkten Blick entspricht, wobei die Augenöffnungsweite (106) einen aktuell erfassten Abstand zwischen den Augenlidern eines Auges (110) repräsentiert,
wobei das Verfahren (500) die folgenden Schritte aufweist:
Ermitteln (502) eines Blinzelereignisses (102) unter Verwendung zumindest eines Blinzelgrenzwerts (116), wobei das Blinzelereignis (102) ermittelt wird, wenn ein, einen Wert der Augenöffnungsweite (106) abbildender Öffnungsweitenwert (112) kleiner ist, als der Blinzelgrenzwert (116); und
wobei im Schritt (502) des Ermittelns ein Mittelblinzelereignis (302) ermittelt wird, wenn der Öffnungsweitenwert (112) kleiner ist, als ein Mittelblinzelgrenzwert, und ein Tiefblinzelereignis (304) ermittelt wird, wenn der Öffnungsweitenwert (112) kleiner ist, als ein Tiefblinzelgrenzwert, wobei ferner ein Tiefblinzelereignis (304) ermittelt wird, wenn ein auf das Ermitteln (502) des Mittelblinzelereignisses (302) nachfolgender Öffnungsweitenwert (112) kleiner als der Tiefblinzelgrenzwert ist und
Bestimmen (504) eines Instrumentenblicks (104) unter Verwendung einer Maximalblinzeldauer (120),
wobei im Schritt (504) des Bestimmens der Instrumentenblick (104) bestimmt wird, wenn das Mittelblinzelereignis (302) länger als die Maximalblinzeldauer (120) ermittelt wird, und wobei kein Instrumentenblick (104) bestimmt wird, wenn das Tiefblinzelereignis (304) länger als die Maximalblinzeldauer (120) ermittelt wird,
insbesondere wobei die Schritte (502, 504) des Verfahrens erneut ausgeführt werden, wenn nachfolgend auf das Bestimmen (504) des Instrumentenblicks (104) der Öffnungsweitenwert (112) größer als ein Offengrenzwert (122) ist.

2. Verfahren (500) gemäß einem der vorangegangenen Ansprüche, mit einem Schritt des Speicherns, wobei ein zeitlicher Verlauf des Öffnungsweitenwerts (112) als ein Blinzelereignisverlauf gespeichert wird, wenn nachfolgend auf das Ermitteln (502) des Blinzelereignisses (102) der Öffnungsweitenwert (112) größer als der Blinzelgrenzwert (116) ist, wobei insbesondere nachfolgend auf den Schritt des Speicherns die Schritte (502, 504) des Verfahrens (500) erneut ausgeführt werden.

3. Verfahren (500) gemäß Anspruch 2, mit einem Schritt des Interpolierens, bei dem die Öffnungsweitenwerte (112) des Blinzelereignisverlaufs zu einer Kurve (400) verbunden werden.

4. Verfahren (500) gemäß Anspruch 3, bei dem im Schritt des Interpolierens die Kurve (400) unter Verwendung einer vorbestimmten Kurvenform (418) approximiert wird.

5. Verfahren (500) gemäß einem der Ansprüche 3 bis 4, bei dem im Schritt des Interpolierens zumindest ein Abweichungswert mindestens eines der Öffnungsweitenwerte (112) von der Kurve (400) bestimmt wird.

6. Verfahren (500) gemäß Anspruch 5, mit einem Schritt des Plausibilisierens, in dem der Blinzelereignisverlauf (400) verworfen wird, wenn der zumindest eine Abweichungswert oder ein davon abgeleiteter Wert größer als ein Abweichungsgrenzwert ist.

7. Verfahren (500) gemäß einem der vorangegangenen Ansprüche, mit einem Schritt des Veränderns, in dem der Blinzelgrenzwert (116) und/oder der Offengrenzwert (122) unter Verwendung eines Bezugsniveaus (126) für die Augenöffnungsweite (106) angepasst wird, wobei das Bezugsniveau (126) die Augenöffnungsweite (106) repräsentiert, wenn kein Blinzelereignis (102) und/oder Instrumentenblick (104) vorliegt.

8. Vorrichtung (100) umfassend eine Ermittlungseinrichtung (114) zur Ermittlung des Öffnungswertes und eine Bestimmungseinrichtung (118) zur Bestimmung des Instrumentenblicks, die eingerichtet ist, das Verfahren (500) gemäß einem der vorangegangenen Ansprüche auszuführen oder durchzuführen.

9. Computerprogramm, das bei Ausführung des Programms durch eine Vorrichtung nach Anspruch 8 dazu eingerichtet ist, das Verfahren (500) gemäß einem der vorangegangenen Ansprüche auszuführen.

10. Maschinenlesbares Speichermedium, auf dem das Computerprogramm nach Anspruch 9 gespeichert ist.

## Claims

1. Method (500) for distinguishing between blinking events (102) and instrument glances (104) with the aid of an apparatus and using an extent to which the eye is open (106), wherein an instrument glance (104) corresponds to a lowered gaze, wherein the extent to which the eye is open (106) represents a currently detected distance between the eyelids of an eye (110), wherein the method (500) includes the following steps:
ascertaining (502) a blinking event (102) using at least one blinking limit value (116), wherein the blinking event (102) is ascertained if a value for the extent of opening (112), mapping a value of the extent to which the eye is open (106), is less than the blinking limit value (116); and
wherein in the ascertainment step (502) a mid blinking event (302) is ascertained if the value for the extent of opening (112) is less than a mid blinking limit value and a low blinking event (304) is ascertained if the value for the extent of opening (112) is less than a low blinking limit value, wherein, further, a low blinking event (304) is ascertained if a value for the extent of opening (112) following the ascertainment (502) of the mid blinking event (302) is less than the low blinking limit value, and
determining (504) an instrument glance (104) using a maximum blinking duration (120),
wherein the instrument glance (104) is determined in the determination step (504) if the mid blinking event (302) is ascertained for a period longer than the maximum blinking duration (120) and wherein no instrument glance (104) is determined if the low blinking event (304) is ascertained for a period longer than the maximum blinking duration (120),
more particularly wherein the steps (502, 504) of the method are carried out again if the value for the extent of opening (112) is greater than an open limit value (122) following the determination (504) of the instrument glance (104).

2. Method (500) according to any one of the preceding claims, comprising a storing step, wherein a time profile of the value for the extent of opening (112) is stored as a blinking event profile if, following the ascertainment (502) of the blinking event (102), the value for the extent of opening (112) is greater than the blinking limit value (116), wherein, more particularly following the step of storing, the steps (502, 504) of the method (500) are carried out again.

3. Method (500) according to Claim 2, comprising an interpolation step, in which the values for the extent of opening (112) of the blinking event profile are connected to form a curve (400).

4. Method (500) according to Claim 3, wherein the curve (400) is approximated using a predetermined curve form (418) in the interpolation step.

5. Method (500) according to either of Claims 3 and 4, wherein at least one deviation value of at least one of the values for the extent of opening (112) from the curve (400) is determined in the interpolation step.

6. Method (500) according to Claim 5, comprising a plausibility-check step, in which the blinking event profile (400) is discarded if the at least one deviation value or a value derived therefrom is greater than a deviation limit value.

7. Method (500) according to any one of the preceding claims, comprising a modification step, in which the blinking limit value (116) and/or the open limit value (122) is adapted using a reference level (126) for the extent to which the eye is open (106), wherein the reference level (126) represents the extent to which the eye is open (106) if no blinking event (102) and/or instrument glance (104) is present.

8. Apparatus (100), comprising an ascertainment device (114) for ascertaining the opening value and a determination device (118) for determining the instrument glance, configured to carry out or perform the method (500) according to any one of the preceding claims.

9. Computer program configured to execute the method (500) according to any one of the preceding claims when said program is executed by an apparatus according to Claim 8.

10. Machine-readable storage medium, on which the computer program according to Claim 9 is stored.

## Revendications

1. Procédé (500) pour effectuer une distinction entre des événements de clignement (102) et des coups d'oeil à des instruments (104) à l'aide d'un dispositif en utilisant une largeur d'ouverture de l'oeil (106), un coup d'oeil à des instruments (104) correspondant à un coup d'oeil abaissé, la largeur de l'ouverture de l'oeil (106) représentant une distance actuellement détectée entre les paupières d'un oeil (110), le procédé (500) présentant les étapes suivantes :
détection (502) d'un événement de clignement (102) en utilisant au moins une valeur limite de clignement (116), l'événement de clignement (102) étant détecté lorsqu'une valeur de largeur d'ouverture (112) reproduisant une valeur de la largeur d'ouverture de l'oeil (106) est inférieure à la valeur limite de clignement (116) ; et
à l'étape (502) de détection, un événement de clignement moyen (302) est détecté lorsque la valeur de largeur d'ouverture (112) est inférieure à une valeur limite de clignement moyen et un événement de clignement bas (304) est détecté lorsque la valeur de largeur d'ouverture (112) est inférieure à une valeur limite de clignement bas, un événement de clignement bas (304) étant en outre détecté lorsqu'une valeur de largeur d'ouverture (112) suivant la détection (502) de l'événement de clignement moyen (302) est inférieure à la valeur limite de clignement bas et détermination (504) d'un coup d'oeil à des instruments (104) en utilisant une durée de clignement maximale (120),
à l'étape (504) de détermination du coup d'oeil à des instruments (104), on détermine si l'événement de clignement moyen (302) est détecté comme étant plus long que la durée de clignement maximale (120), et aucun coup d'oeil à des instruments (104) n'étant déterminé si l'événement de clignement bas (304) est déterminé comme étant plus long que la durée de clignement maximale (120),
en particulier, les étapes (502, 504) du procédé étant effectuées de manière renouvelée si, suite à la détermination (504) du coup d'oeil à des instruments (104), la valeur de largeur d'ouverture (112) est plus grande qu'une valeur limite d'ouverture (122).

2. Procédé (500) selon l'une quelconque des revendications précédentes, comprenant une étape de mémorisation, une allure dans le temps de la valeur de largeur d'ouverture (112) étant mémorisée sous forme de courbe d'événements de clignement lorsque suite à la détection (502) de l'événement de coup d'oeil (102), la valeur de largeur d'ouverture (112) est supérieure à la valeur limite de clignement (116), et notamment, suite à l'étape de mémorisation, les étapes (502, 504) du procédé (500) étant effectuées de manière renouvelée.

3. Procédé (500) selon la revendication 2, comprenant une étape d'interpolation dans laquelle les valeurs de largeur d'ouverture (112) de l'allure des événements de clignement sont reliées pour former une courbe (400).

4. Procédé (500) selon la revendication 3, dans lequel, à l'étape d'interpolation, la courbe (400) est approximée en utilisant une forme de courbe prédéterminée (418).

5. Procédé (500) selon l'une quelconque des revendications 3 et 4, dans lequel, à l'étape d'interpolation, on détermine au moins une valeur d'écart d'au moins l'une des valeurs de largeur d'ouverture (112) par rapport à la courbe (400).

6. Procédé (500) selon la revendication 5, comprenant une étape de plausibilité, dans laquelle l'allure des événements de clignement (400) est rejetée si l'au moins une valeur d'écart ou une valeur dérivée de celle-ci est supérieure à une valeur limite d'écart.

7. Procédé (500) selon l'une quelconque des revendications précédentes, comprenant une étape de modification, dans laquelle la valeur limite de clignement (116) et/ou la valeur limite d'ouverture (122) sont adaptées en utilisant un niveau de référence (126) pour la largeur d'ouverture de l'oeil (106), le niveau de référence (126) représentant la largeur d'ouverture de l'oeil (106) lorsqu'il n'y a aucun événement de clignement (102) et/ou de coup d'oeil à des instruments (104).

8. Dispositif (100) comprenant un dispositif de détection (114) pour déterminer la valeur d'ouverture et un dispositif de détermination (118) pour déterminer le coup d'oeil à des instruments, qui est prévu pour réaliser ou mettre en oeuvre le procédé (500) selon l'une quelconque des revendications précédentes.

9. Programme informatique qui, lors de l'exécution du programme par un dispositif selon la revendication 8, est prévu pour mettre en oeuvre le procédé (500) selon l'une quelconque des revendications précédentes.

10. Support de mémoire lisible par machine sur lequel est mémorisé le programme informatique selon la revendication 9.
